Europäisches Patentamt
European Patent Office
Office européen des brevets

(11) Publication number: 0 289 070 A1

# EUROPEAN PATENT APPLICATION

(21) Application number: 88200648.9

(22) Date of filing: 06.04.88

(51) Int. Cl.4: C07C 91/30 , C07C 97/00 , C07C 103/50 , C07D 207/335 , C07D 231/12 , A61K 31/135 , A61K 31/16 , A61K 31/40 , A61K 31/415

(30) Priority: 10.04.87 NL 8700842

(43) Date of publication of application: 02.11.88 Bulletin 88/44

(84) Designated Contracting States: AT BE CH DE ES FR GB GR IT LI LU NL SE

(71) Applicant: DUPHAR INTERNATIONAL RESEARCH B.V
C.J. van Houtenlaan 36
NL-1381 CP Weesp(NL)

(72) Inventor: Van Wijngaarden, Ineke
c/o Octrooibureau Zoan B.V. P.O. Box 140
NL-1380 AC Weesp(NL)
Inventor: Den Hartog, Jacobus A. J.
c/o Octrooibureau Zoan B.V. P.O. Box 140
NL-1380 AC Weesp(NL)
Inventor: Tulp, Martinus T. M.
c/o Octrooibureau Zoan B.V. P.O. Box 140
NL-1380 AC Weesp(NL)
Inventor: Lobbezoo, Marinus W.
c/o Octrooibureau Zoan B.V. P.O. Box 140
NL-1380 AC Weesp(NL)

(74) Representative: Muis, Maarten et al
OCTROOIBUREAU ZOAN B.V. P.O. Box 140
NL-1380 AC Weesp(NL)

(54) **Tertiary arylethyl amine derivatives having opiate-antagonistic activity.**

(57) The invention relates to a group of tertiary arylethyl amine derivatives having opiate-antagonistic activity. The compounds have the general formula 1

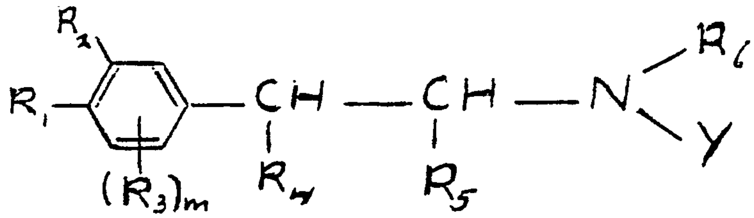

(1)

wherein

EP 0 289 070 A1

$R_1$ is hydrogen, an optionally esterified hydroxyl group or mercapto group, a group $-NHR_9$ or $-CONHR_9$, wherein $R_9$ is hydrogen, alkyl having 1-6 C-atoms or alkylcarbonyl having 1-7 C-atoms;
$R_2$ is hydrogen or, when $R_1$ is hydrogen, one of the other meanings of $R_1$, or
$R_1$ and $R_2$ together with the 2 carbon atoms of the benzene ring constitute a heterocyclic group which consists of five or six ring atoms and which comprises a group -NH-and, optionally may comprise an oxygen atom, sulphur atom or nitrogen atom as a second hetero atom;
$R_3$ is hydrogen, alkyl, alkoxy or alkylthio having 1-4 C-atoms, amino, mono-or dialkylamino having 1-4 C-atoms per alkyl group, hydroxyalkyl, alkyl-, alkylamino-or alkoxycarbonyl having 1-4 C-atoms in the alkyl group, nitro, cyano, halogen, trifluoromethyl, trifluoromethoxy, alkylsulphonyl having 1-4 C-atoms, or aminosulphonyl;
m has the value 1, 2 or 3;
$R_4$ is hydrogen, alkyl or alkoxy having 1-3 C-atoms, or hydroxyl;
$R_5$ is hydrogen; alkyl, phenylalkyl, hydroxyalkyl, methoxyalkyl, alkylcarbonyl, alkoxycarbonyl or alkylaminocarbonyl having 1-6 C-atoms in the optionally branched alkyl group;
$R_6$ is straight or branched alkyl, alkenyl or cycloalkylalkyl or cycloalkyl, having at most 8 C-atoms;
Y is a group $R_7$-X-$R_8$, wherein $R_7$ is a straight or branched alkylene chain having 3-8 C-atoms with at least 3 C-atoms between the nitrogen atom and group X; X is the carbonyl group or ketalised carbonyl group, or the group CHOH, $CHC_6H_5$, $CH_2$, -CONH-or $-CO-NCH_3$ or an oxygen atom or sulphur atom; and $R_8$ is an alkyl group, cycloalkyl group or cycloalkylalkyl group having at most 10 C-atoms a phenyl group or phenylalkyl group having 1-4 C-atoms in the alkyl group, which groups $R_8$ can be substituted with one or more groups $R_3$; or Y is a group of the formula 2a-2e

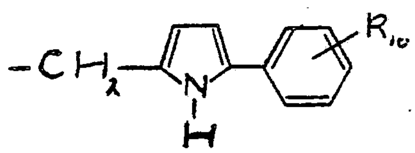

2a

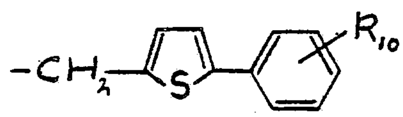

2b

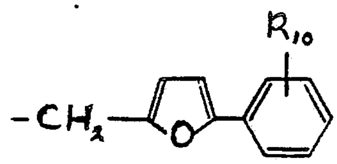

2c

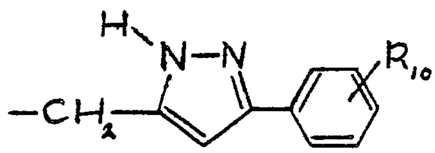

2d

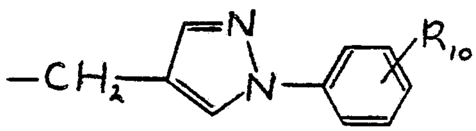

2e

wherein $R_{10}$ may have the meanings given for $R_3$.

# Tertiary arylethyl amine derivatives having opiate-antagonistic activity.

The invention relates to tertiary arylethyl amine derivatives having opiate-antagonistic properties. The invention also relates to the salts and prodrugs of these compounds, to a method of preparing the active compounds, and to pharmaceutical compositions comprising at least one of these new compounds or a salt or prodrug thereof as the active substance.

It is known that in animals and man receptors are present with which endogenous opioids, i.e. opioids which naturally occur in the body, for example the enkephalines, interact. Although the activity of these endogenous opioids can be very favourable in a number of cases, a great number of conditions are known in which the effects of these endogenous opioids are just particularly negative. Compounds which show an antagonistic activity against these endogenous opioids may, therefore be used in the treatment of a number of syndromes in man. Such opiate antagonists may also be used to counteract the effects of exogenous opiates, for example, morphine. For these purposes, substances are preferably used which have a pure opiate-antagonistic effect without an agonistic component in order to avoid the danger of undesired addictive properties associated with opiate-agonism.

A few compounds are known which have a pure opiate-antagonistic activity, notably naloxone, naltrexone and nalmephene. Structurally, these compounds are closely related to each other and to the known exogenous opiate-agonist morphine.

It has now been found that the compounds of the general formula 1:

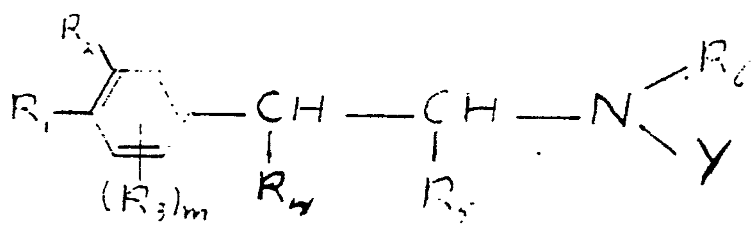

(1)

and the salts thereof have a strong, pure opiate-antagonistic activity.

In formula 1 the symbols have the following meanings:

$R_1$ is hydrogen, an optionally esterified hydroxyl group or mercapto group, a group $-NHR_9$ or $-CONHR_9$, wherein $R_9$ is hydrogen, alkyl having 1-6 C-atoms or alkylcarbonyl having 1-7 C-atoms;

$R_2$ is hydrogen or, when $R_1$ is hydrogen, one of the other meanings of $R_1$, or

$R_1$ and $R_2$ together with the 2 carbon atoms of the benzene ring constitute a heterocyclic group which consists of five or six ring atoms and which comprises a group -NH-and, optionally may comprise an oxygen atom, sulphur atom or nitrogen atom as a second hetero atom;

$R_3$ is hydrogen, alkyl, alkoxy or alkylthio having 1-4 C-atoms, amino, mono-or dialkylamino having 1-4 C-atoms per alkyl group, hydroxyalkyl, alkyl-, alkylamino-or alkoxycarbonyl having 1-4 C-atoms in the alkyl group, nitro, cyano, halogen, trifluoromethyl, trifluoromethoxy, alkylsulphonyl having 1-4 C-atoms, or aminosulphonyl;

m has the value 1, 2 or 3;

$R_4$ is hydrogen, alkyl or alkoxy having 1-3 C-atoms, or hydroxyl;

$R_5$ is hydrogen; alkyl, phenylalkyl, hydroxyalkyl, methoxyalkyl, alkylcarbonyl, alkoxycarbonyl or alkylaminocarbo nyl having 1-6 C-atoms in the optionally branched alkyl group;

$R_6$ is straight or branched alkyl, alkenyl or cycloalkylalkyl or cycloalkyl, having at most 8 C-atoms;

Y is a group $R_7$-X-$R_8$, wherein $R_7$ is a straight or branched alkylene chain having 3-8 C-atoms with at least 3 C-atoms between the nitrogen atom and group X; X is the carbonyl group or ketalised carbonyl group, or the group $>$CHOH, $>CHC_6H_5$, $>CH_2$, -CONH-or -CO-N($CH_3$) or an oxygen atom or sulphur atom; and $R_8$ is an alkyl group, cycloalkyl group or cycloalkylalkyl group having at most 10 C-atoms a phenyl group or phenylalkyl group having 1-4 C-atoms in the alkyl group, which groups $R_8$ can be substituted with one or more groups $R_3$; or Y is a group of the formula 2a-2e

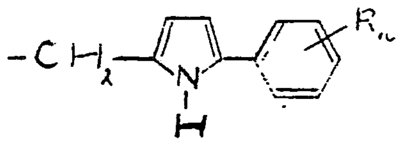 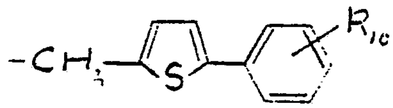 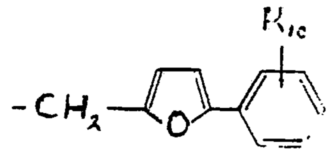

2a 2b 2c

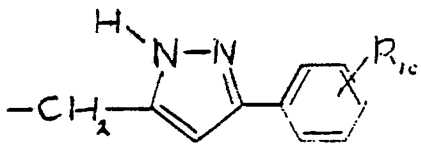 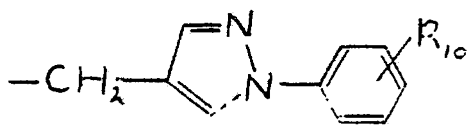

2d 2e

wherein $R_{10}$ may have the meanings given for $R_3$.

Although some of these compounds (i.e. compounds wherein $R_1$ or $R_2$ is hydroxyl) are in the scope of Netherlands patent application no. 7404733 none of the described known compounds has an hydroxylated phenyl group.

The compounds which on the basis of their properties are to be preferred are compounds of formula 1 wherein the symbols have the following meanings, and salts and prodrugs thereof:

$R_1$ is hydrogen, optionally esterified hydroxyl or aminocarbonyl;

$R_2$ is hydrogen or, when $R_1$ is hydrogen, optionally esterified hydroxyl or aminocarbonyl;

$R_3$ is hydrogen, methyl, methoxy or halogen in the ortho position with respect to the group $-CHR_4-CHR_5-NR_6Y$;

m has the value 1;

$R_4$ is hydrogen or hydroxyl;

$R_5$ is alkyl having 1-3 C-atoms or phenyl ethyl;

$R_6$ is alkyl having 1-4 C-atoms, propenyl, butenyl or cyclopropylmethyl;

Y is the group $R_7$-X-$R_8$, wherein $R_7$ is trimethylene, X is carbonyl, >CHOH, -CONH-, $-CH_2-$or an oxygen atom, and $R_8$ is cyclohexyl, phenyl or halogen-substituted phenyl; or Y is a group of formula 2a, 2d or 2e, wherein $R_{10}$ is hydrogen or halogen.

Compounds according to the invention which are to be preferred in particular are:

1. 1-cyclohexyl-4-[propyl[2-(4-hydroxyphenyl)-1-methylethyl]amino]-1-butanone,
2. 1-cyclohexyl-4-[propyl[2-(4-hydroxyphenyl)-1-methylethyl]amino]-1-butanol,
3. 4-[2-[propyl(4-cyclohexyl-4-oxobutyl)amino]2-methylethyl]benzamide,
4. 4-[2-[propyl(4-cyclohexyl-4-hydroxybutyl)amino]-2-methylethyl]benzamide,
5. 1-cyclohexyl-4-[propyl[2-(3-hydroxyphenyl)-1-methylethyl]amino]-1-butanone,
6. 1-cyclohexyl-4-[propyl[2-(3-hydroxyphenyl)-1-methylethyl]amino]-1-butanol,
7. 1-cyclohexyl-4-[propyl[2-(4-benzoyloxyphenyl)-1-methylethyl]amino]-1-butanone,
8. 1-cyclohexyl-4-[propyl[2-(4-acetoxyphenyl)-1-methylethyl]amino]-1-butanone,
9. ammonium 4-[2-[propyl(4-cyclohexyl-4-oxobutyl)amino]-2-methylethyl]phenylphosphate,
10. N-(4-chlorophenyl) 4-[2-[propyl(4-cyclohexyl-4-oxobutyl)amino]-2-methylethyl]phenylcarbamate,
11. N-acetyl 4-[2-[propyl(4-cyclohexyl-4-oxobutyl)amino]2-methylethyl]phenylcarbamate,
12. 1-cyclohexyl-4-[propyl[2-(4-hydroxy-2-methylphenyl)-1-methylethyl]amino]-1-butanone,
13. 1-cyclohexyl-4-[propyl[2-(4-hydroxy-2-methoxyphenyl)-1-methylethyl]amino]-1-butanone,
14. 1-cyclohexyl-4-[propyl[2-(4-hydroxyphenyl)-1-methyl-2-hydroxyethyl]amino]-1-butanone,
15. 1-cyclohexyl-4-[propyl[2-(4-hydroxyphenyl)-1-ethylethyl]amino]-1-butanone,
16. 1-cyclohexyl-4-[propyl[2-(4-hydroxyphenyl)-1-ethylethyl]amino]-1-butanol,
17. 1-cyclohexyl-4-[propyl[2-(4-hydroxyphenyl)-1-propylethyl]amino]-1-butanone,
18. 1-cyclohexyl-4-[propyl[2-(4-hydroxyphenyl)-1-propylethyl]amino]-1-butanol,
19. 1-cyclohexyl-4-[propyl[1-(2-phenylethyl)-2-(4-hydroxyphenyl)ethyl]amino]-1-butanone,
20. 1-cyclohexyl-4-[methyl[2-(4-hydroxyphenyl)-1-methylethyl]amino]-1-butanone,
21. 1-cyclohexyl-4-[ethyl[2-(4-hydroxyphenyl)-1-methylethyl]amino]-1-butanone,

22. 1-cyclohexyl-4-[ethyl[2-(4-hydroxyphenyl)-1-methylethyl]amino]-1-butanol,
23. 1-cyclohexyl-4-[butyl[2-(4-hydroxyphenyl)-1-methylethyl]amino]-1-butanone,
24. 1-cyclohexyl-4-[2-propenyl[2-(4-hydroxyphenyl)-1-methylethyl]amino]-1-butanone,
25. 1-cyclohexyl-4-[2-propenyl[2-(4-hydroxyphenyl)-1-methylethyl]amino]-1-butanol,
26. 1-cyclohexyl-4-[3-butenyl[2-(4-hydroxyphenyl)-1-methylethyl]amino]-1-butanone,
27. 1-cyclohexyl-4-[3-butenyl[2-(4-hydroxyphenyl)-1-methylethyl]amino]-1-butanol,
28. 1-cyclohexyl-4-[cyclopropylmethyl[2-(4-hydroxyphenyl)-1-methylethyl]amino]-1-butanone,
29. 1-cyclohexyl-4-[cyclopropylmethyl[2-(4-hydroxyphenyl)-1-methylethyl]amino]-1-butanol,
30. 4-[2-[propyl(4-cyclohexylbutyl)amino]-2-methylethyl]phenol,
31. N-cyclohexyl-4-[propyl[2-(4-hydroxyphenyl)-1-methylethyl]amino]butyramide,
32. 4-[2-[propyl(3-phenoxypropyl)amino]-2-methylethyl]phenol,
33. 1-phenyl-4-[propyl[2-(4-hydroxyphenyl)-1-methylethyl]amino]-1-butanone,
34. 1-phenyl-4-[propyl[2-(4-hydroxyphenyl)-1-methylethyl]amino]-1-butanol,
35. 1-(4-fluorophenyl)-4-[propyl[2-(4-hydroxyphenyl)-1-methylethyl]amino]-1-butanone,
36. N-phenyl-4-[propyl[2-(4-hydroxyphenyl)-1-methylethyl]amino]butyramide,
37. 1-cyclohexyl-4-[propyl[2-(5-indolyl)-1-methylethyl]amino]-1-butanone,
38. 4-[2-[propyl[5-(4-fluorophenyl)pyrrol-2-yl-methyl]amino]-2-methylethyl]phenol,
39. 4-[2-[propyl(5-phenylpyrazol-3-yl-methyl)amino]-2-methylethyl]phenol,
40. 4-[2-[propyl[1-(4-chlorophenyl)pyrazol-4-yl-methyl]amino]-2-methylethyl]phenol.

Examples of suitable acids with which the compounds according to the invention can form pharmaceutically acceptable acid addition salts are hydrochloric acid, sulphuric acid, phosphoric acid, nitric acid, and organic acids, for example, citric acid, fumaric acid, maleic acid, tartaric acid, acetic acid, benzoic acid, p-toluene sulphonic acid, methane sulphonic acid, naphthalene sulphonic acid, and the like.

Examples of suitable bases with which those compounds according to the invention which comprise an acid group can form pharmaceutically acceptable salts are ammonium hydroxyde, sodium hydroxide, potassium hydroxide and calcium hydroxide.

Prodrugs denote derivatives of the compounds of formula 1 which as such are inactive and which, after administration into the body, are converted into an active substance of formula 1.

In those cases in which the group $R_4$ and/or $R_5$ in compounds according to formula 1 have a meaning other than hydrogen, the carbon atoms to which $R_4$ and/or $R_5$ are bound are chiral centres. In so far as chiral centres are concerned, the invention relates to the various enantiomers of the compound of formula 1 and to the racemates.

On the basis of their opiate-antagonistic activity, compounds according to the invention are extremely suitable for the treatment of those diseases and conditions in man in which endogenous opioids play a part. Examples are: schizophrenia, depression, epilepsy and other diseases associated with the central nervous system, shock, stroke and other disorders associated with the cardiovascular system, ulcers, obesity, respiratory disorders and several types of tumours, especially neuroblastoma. They may also be used for the treatment of patients after an overdose of exogenous opiates, to terminate anesthesia with exogenous opiates and as an auxiliary agent to prevent recidivism in previous addicts of exogenous opiates.
The compounds according to the invention have been tested for the activities hereinafter in a number of test models. Naloxone was used as a reference substance.

1. Opiate-(ant)agonistic activity in vitro.

1.1 Affinity to opiate receptors.

The affinity to (mainly $\mu$-type) opiate receptors was determined by studying the displacement of [$^3$H]-naloxone in homogenates of rat brains (Pert and Snyder, Molecular Pharmacology, 10, 868-879 (1974)). The results were expressed in $K_i$-values.

1.2 Opiate-(ant)agonistic activity on the isolated guinea pig ileum and mouse vas deferens.

The opiate-antagonistic activity was determined by studying the antagonism of the effect of the agonists morphine and ethylketazocine on the electrically stimulated guinea pig ileum ($\mu$-type and (substantially) k-type opiate antagonism, respectively) and the antagonism of the effect of the agonist leucine-enkephaline on the electrically stimulated mouse vas deferens ($\delta$-type opiate antagonism).

The results were expressed in $pA_2$ values.

In order to establish opiate-agonistic activity, if any, the effect of the test compounds on the electrically stimulated guinea pig ileum and mouse vas deferens was determined. In order to establish whether a found effect, if any, was caused by opiate agonism, the reversal of this effect, if any, by the antagonist naloxone was studied. The above experiments were carried out as described in Magnan et al, Naunyn Schmiedeberg's Arch. Pharmacol. 319, 197-205 (1982), or, for the experiments with ethylketazocine, entirely analogously to the experiments with morphine described in the said publication.

2. Opiate-(ant)agonistic activity in vivo.

Opiate-antagonistic activity in vivo was determined by studying the antagonism of morphine-induced analgesia in mice, measured according to Bianchi and Francheschini, Br. J. Pharmacol. Chemother. 9, 280-284 (1954). Test compounds were administered subcutaneously (sc) or orally (po) in a series of dosages, using five animals for each dose, and the results were expressed in $ED_{50}$ values. In order to establish an opiate-agonistic activity, if any, possible analgetic activity was investigated for the highest dose used in the antagonistic test.

The compounds according to the invention show a structural relationship with the specific muscarinolytics known from the Netherlands Patent Application 7404733. For that reason the affinity to muscarine receptors was determined in addition to that for opiate receptors.

3. Possible anticholinergic side effect in vitro; affinity to cholinergic muscarine receptors.

The affinity to cholinergic muscarine receptors was determined by studying the displacement of [$^3$H] Quinuclidinyl benzilate (QNB) in homogenates of rat brains (Yamamura and Snyder, Proc. Natl. Acad. Sci. U.S.A. 71, 1725 (1974)). The results were expressed in $K_i$ values.

From the tests as described sub 1 and 3 it can be concluded that the structural characteristics which lead to a good opiate-antagonistic and a good muscarinolytic activity, respectively, diverge considerably. This is illustrated in Table 1 with reference to a few compounds. Moreover it must be noted that for the racemic compound b recorded in Table 1, of which the pure R and S isomers were available, the opiate-antagonistic activity was found substantially entirely in the R isomer (c) while the muscarinolytic activity was found substantially exclusively in the S isomer (d).

TABLE 1

Affinity to opiate and cholinergic muscarine receptors of a few compounds from Netherlands Patent Application 7404733 (e to g inclusive), a few compounds of formula 1 according to the present invention (a to d inclusive) and two reference substances.

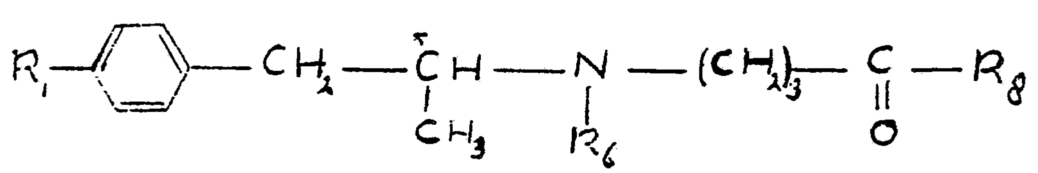

| Comp. | $R_1$ | $R_6$ | $R_8$ | isomer | Affinity to | |
|---|---|---|---|---|---|---|
| | | | | | opiate recept. 1) | muscarine receptors 2) |
| naloxone | | | | | 1.5 | 34000 |
| a | hydroxy | propyl | cyclohexyl | rac. | 0.8 | 5.2 |
| b | " | ethyl | " | rac. | 25 | 9.6 |
| c | " | " | " | R | 18 | 450 |
| d | " | " | " | S | 200 | 4.1 |
| e | methoxy | " | n-propyl | rac. | 5200 | 28 |
| f | chloro | " | cyclohexyl | rac. | 3750 | 8.3 |
| g | methoxy | " | " | rac. | 1400 | 2.7 |
| atropine | | | | | 16200 | 1.4 |

1) [$^3$H] naloxone displacement expressed in $K_i$
2) [$^3$H] QNB displacement expressed in $K_i$

The new compounds according to the invention and the salts and prodrugs thereof can be prepared in a manner known for the synthesis of analogous compounds.

The invention therefore also relates to a method of preparing new tertiary arylethyl amine derivatives of formula 1, wherein the symbols have the meanings given hereinbefore, and the salts and prodrugs thereof.

Suitable methods of preparing the compounds of formula 1 as a rule comprise the reaction of a secondary amine of formula 3, or derivatives thereof, with reagents which comprise the group Y, or fragments or derivatives thereof.

Depending on the meanings of the symbols, the compounds of formula 1 can be obtained inter alia by means of any of the following methods.

Compounds of formula 1, wherein Y = $R_7$ -X - $R_8$, may be obtained, for example, by converting an amine of formula 3

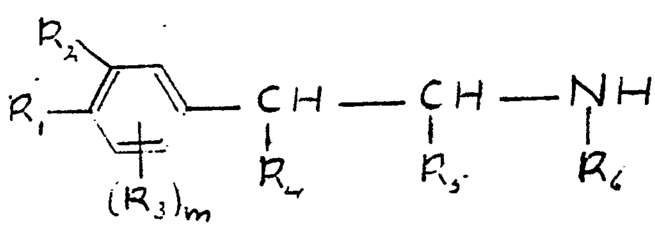
(3)

wherein $R_1$ -$R_6$ and m have the above meanings, with a compound of the general formula 4

L—$R_7$—X'—$R_8$ (4)

wherein $R_7$ and $R_8$ have the above meanings, L is a halogen atom or a tosyloxy group, and X' is a carbonyl group, 1,3-dioxolane group, a >CH-phenyl group or a methylene group, or an oxygen atom or a sulphur atom.

The reaction is preferably carried out in an inert solvent, for example, dimethyl formamide or acetonitrile, or without a solvent, at a temperature of 0-180°C, preferably 20-80°C for 1-48 hours; a base,

for example, triethylamine or sodium carbonate, may be added to the reaction mixture or an excess of the amine (3) may be used; furthermore, in case L is a chlorine atom, NaI may be added to the reaction mixture as a catalyst.

If desired, the resulting compounds of formula 1, wherein Y = $R_7$ -X - $R_8$ and X is a 1,3-dioxolane group may be converted into the analogous compounds, wherein X is a carbonyl group, by treating with a dilute acid, for example, hydrochloric acid.

The resulting compounds of formula 1, wherein Y = $R_7$ -X - $R_8$, wherein X is a carbonyl group may then be further converted in known manner (J. Am. Chem. Soc. 93, 2897, (1971)) into analogous compounds, wherein X is a $\gt$CHOH group, by treating with a reduction agent, for example, $NaCNBH_4$.

Compounds of formula 1, wherein Y is the group $R_7$ -X - $R_8$, wherein X is the group CONH or $CONCH_3$, can be obtained by converting an ester of formula 5

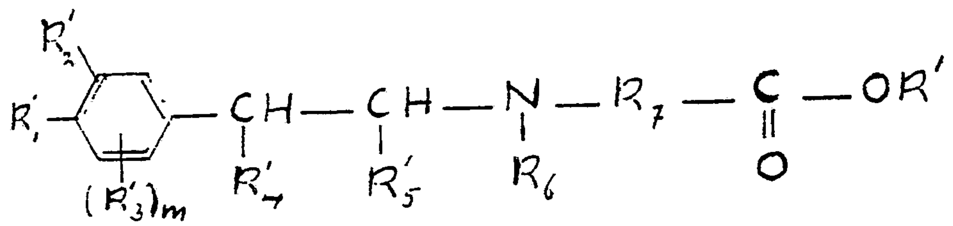
(5)

wherein $R_1'$ -$R_5'$ have the meanings mentioned for $R_1$ -$R_5$, with the proviso that reactive hydrogen atoms present therein are replaced by a protective group, $R_6$, $R_7$ and m have the above meanings and R' is a lower alkyl group, with an amine of formula 6 or 7

$H_2N - R_8$ (6) $CH_3NH - R_8$ (7)

wherein $R_8$ has the above meaning, and then optionally removing the protective group(s).

The reaction of a compound of formula 5 with an amine (6) or (7) is preferably carried out in a inert solvent, for example, toluene or dimethyl sulphoxide, or without a solvent, in the presence of a base, for example, sodium hydride or sodium methoxide, at a temperature of 0 to 100°C, preferably room temperature, for 1-48 hours.

The esters of formula 5 used as starting substances may be obtained by converting an amine of formula 8

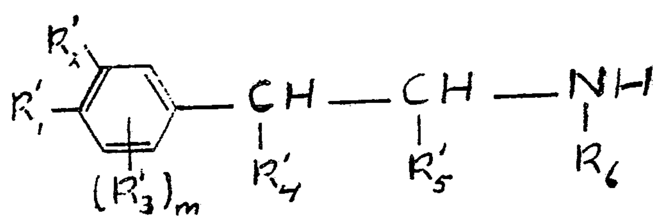
(8)

wherein $R_1'$ -$R_5'$, m and $R_6$ have the above meanings, with a compound of formula 9

$L{-}R_7{-}\overset{\overset{O}{\|}}{C}{-}OR'$ (9)

wherein R', $R_7$ and L have the above meanings.

The reaction of a compound of formula 8 with a compound of formula 9 is carried out in a manner known for analogous compounds (Patai, "The chemistry of the amino group", pp. 45-55, Interscience Publishers, New York, 1968).

Compounds of formula 1, wherein Y is a group of formulae 2a -2c, can be obtained, for example, by a so-called Mannich reaction. In this reaction the adduct which is formed after treating an amine of formula 3 with formaldehyde, is converted with a 2-phenylpyrrole, 2-phenylthiophene or 2-phenylfuran derivative.

The reaction is carried out in an organic solvent, preferably a protic solvent, and may optionally be accelerated by the addition of an organic or inorganic acid as a catalyst. The reaction temperature is preferably between room temperature and the boiling-point of the solvent used.

Compounds of formula 1, wherein Y is a group of formula 2d or 2e, can be obtained, for example, by converting an amine of formula 3 with a compound of formula 10

L—Y (10)

wherein Y is the group 2d or 2e and L is a halogen atom or a tosyloxy group.

The reaction of a compound of formula 3 with a compound of formula 10 is carried out analogously to the above-described reaction of a compound of formula 3 with a compound of formula 4.

The amines of formula 3, wherein $R_1$ -$R_6$ and m have the above meanings, used as starting substances can be obtained, for example, by converting a ketone of formula 11

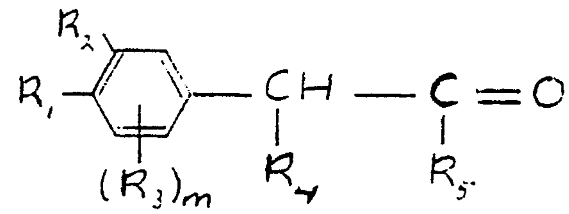

(11)

in the presence of a reduction agent in known manner (Org. React. 4, 174 (1948) and J. Am. Chem. Soc. 93, 2897, (1971)) with an amine of formula 12

$H_2N—R_6$ (12)

The ketones of formula 11 are partly known compounds and, as far as they are new compounds, these can be obtained in a manner known for the preparation of analogous ketones.

The amines of formula 3 can further be obtained by monoalkylating an amine of formula 13

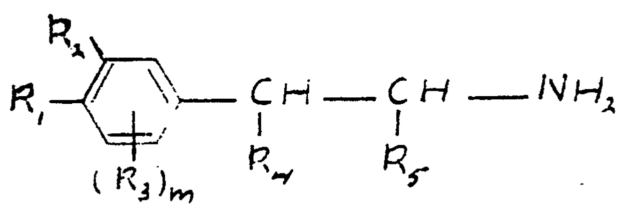

(13)

in known manner (Patai, "The Chemistry of the amino group", pp. 45-55, Interscience Publishers, New York, 1968), with a compound (14)

$L—R_6$ (14)

wherein L is halogen atom or a tosyloxy group. The amines of formula 3 can also be obtained by converting an amine of formula 13 with a carboxylic acid chloride (see inter alia Zabicky, "The chemistry of amides", pp. 73-119, Interscience Publishers, New York, 1956), in such a manner that after reduction with, for example, $LiAlH_4$ (see inter alia Gaylord, "Reduction with complex metal hydrides", pp. 544-594, Interscience Publishers, New York, 1956), the secondary amine (3) is obtained with the desired substi tuent $R_6$.

The amines of formula 13 are partly known compounds and, as far as they are new compounds, these can be obtained in manner known for the preparation of analogous amines.

Within the meanings of $R_1$-$R_{10}$, m, X and Y, a number of chemical conersions known per se, for example, reduction reactions, esterifications, amidations, alkylations, dealkylations, etc. may be used as the last step in the reaction to prepare compounds of formula 1.

The invention will be described in greater detail with reference to the ensuing specific examples. The compounds were obtained as a high-boiling-point oil the boiling-point of which could not be determined as a result of decomposition. The compounds were characterized by means of $^1H$ NMR or $^{13}C$ NMR.

## EXAMPLE I

### 1-cyclohexyl-4-[propyl[2-(4-hydroxyphenyl)-1-methylethyl]amino-1-butanone.

2.92 g (27.5 mmol) of sodium carbonate, 4.13 g (27.5 mmol) of sodium iodide and 6.4 g (27.5 mmol) of 2-(3-chloropropyl)-2-cyclohexyl-1,3-dioxolane were added to a solution of 4.83 g (25 mmol) of propyl[2-(4-hydroxyphenyl)-1-methylethyl]amine in 30 ml of dimethyl formamide and the resulting reaction mixture was stirred for 18 hours at a temperature of 80°C.

After cooling, the reaction mixture was poured on ice and extracted three times with ethyl acetate. After evaporating the organic layer under reduced pressure, the residue was dissolved in a mixture of 30 ml of

dimethyl formamide and 45 ml of 2N hydrochloric acid and stirred for 1 hour so as to split the dioxolane group present. The solution was then extracted three times with diethyl ether, made basic by the addition of concentrated ammonia and extracted three times with ethyl acetate.

The resulting organic layer was washed three times with water and once with concentrated saline, dried on sodium sulphate and evaporated under reduced pressure. The resulting crude product (7.7 g) was purified chromatographically over 200 g of silica gel (Merck, grain size 0.063-0.200 mm) using a mixture of dichloromethane, methanol and concentrated ammonia in the ratio 93:6.5:0.5 as an eluent. After evaporating, 5.5 g (16 mmol) of 1-cyclohexyl-4-[propyl[2-(4-hydroxyphenyl)-1-methylethyl]amino]-1-butanone, i.e. the above-mentioned compound 1, were obtained.

The compounds 3, 5, 12, 13, 14, 15, 17, 19, 20, 21, 23, 24, 26, 28, 30, 32, 33, 35 and 37 mentioned hereinbefore were obtained in an analogous manner.

The following derivatives were prepared from the above-mentioned compounds by chemical conversions known per se: 2, 4, 6, 7, 8, 9, 10, 11, 16, 18, 22, 25, 27, 29 and 34.

EXAMPLE II

N-cyclohexyl-4-[propyl[2-(4-hydroxyphenyl)-1-methylethyl]amino]butyramide.

1.75 g (40 mmol) of sodium hydride (as a 55% dispersion in oil) were added under nitrogen to a mixture of 20 ml of dry dimethyl sulphoxide and 10 ml of dry toluene and stirred at room temperature for 30 minutes. 3.3 g (33 mmol) of cyclohexylamine were added in small portions at 20-25°C while stirring, and the mixture was then stirred at room temperature for 30 minutes. A solution of 9.6 g (30 mmol) of ethyl 4-[propyl-[2-(4-methoxyphenyl)-1-methylethyl]amino]butanoate in 20 ml of dry dimethyl sulphoxide and 10 ml of dry toluene was added dropwise and the mixture was stirred at room temperature for 24 hours.

200 ml of water were added to the reaction mixture, the temperature being kept below 30°C.

The aqueous layer was acidified to pH 5 with 2 N hydrochloric acid, then neutralised (till pH 7 to 8) with sodium bicarbonate and extracted three times with diethyl ether.

The aqueous layer was made basic with 2 N sodium hydroxide and extracted three times with methylene chloride.

The collected methylene chloride layers were washed once with little water, dried on sodium sulphate and evaporated under reduced pressure.

The resulting crude product (8.5 g) was purified chromatographically over 250 g of silica gel (Merck, grain size 0.063-0.200 mm) using a mixture of dichloromethane, methanol and concentrated ammonia in the ratio 92.5:7.0:0.5 as an eluent.

After evaporating the collected fractions under reduced pressure, 7.1 g (19 mmol) of pure product were obtained.

The resulting product was dissolved in 140 ml of dichloromethane. At a temperature between -60 and -50°C a solution of 24 g (95 mmol) of borotribromide in 70 ml of dichloromethane were added dropwise under nitrogen in 30 minutes. The reaction mixture was stirred for another 2 hours, the mixture slowly reaching room temperature.

After cooling again to -50°C, 50 ml of $H_2O$ and then 50 ml of concentrated ammonia were added dropwise. After stirring at room temperature for 2 hours, three extractions with dichloromethane were carried out. The collected organic layers were dried on sodium sulphate and evaporated under reduced pressure. The resulting crude product (6.5 g) was purified chromatographically by means of flash chromatography over 600 g of silica gel (Merck, grain size 0.040-0.063 mm) using a mixture of dichloromethane, methanol and concentrated ammonia in the ratio 93:6.5:0.5 as an eluent.

After evaporating the collected fractions under reduced pressure, 3.7 g (10 mmol) of N-cyclohexyl-4-[propyl[2-(4-hydroxyphenyl)-1-methylethyl]amino]butyramide were obtained (compound 31).

Compound 36 was obtained in an analogous manner.

EXAMPLE III

4-[2-[propyl[5-(4-fluorophenyl)pyrrol-2-yl-methyl]amino]-2-methylethyl]phenol.

0.8 g (10 mmol) of formalin (content 37%), 1 ml of glacial acetic acid and 1.6 g (10 mmol) of 2-(4-fluorophenyl)pyrrole were added to a solution of 1.9 g (10 mmol) of propyl[2-(4-hydroxyphenyl)-1-methylethyl)amine in 80 ml of absolute ethanol.

After stirring at room temperature for 40 hours, the mixture was neutralised by the addition of concentrated ammonia and evaporated under reduced pressure. Water and dilute ammonia were added to the residue and the whole was extracted three times with ethyl acetate. The collected organic layers were dried on sodium sulphate and evaporated under reduced pressure.

The resulting crude product was purified chromato graphically over 200 g of silica gel (Merck, grain size 0.063-0.200 mm) using a mixture of dichloromethane, methanol and concentrated ammonia in the ratio 95.5:4.0:0.5 as an eluent.

After evaporating the collected fractions under reduced pressure, 1.6 g (4 mmol) of 4-[2-[propyl[5-(4-fluorophenyl)pyrrol-2-yl-methyl]amino]-2-methylethyl]phenol were obtained (compound 38).

EXAMPLE IV

4-[2-[propyl(5-phenylpyrazol-3-yl)methylamino]-2-methylethyl]phenol.

1.4 g (16.5 mmol) of sodium carbonate were added to a solution of 1.5 g (7.8 mmol) of propyl[2-(4-hydroxyphenyl)-1-methylethyl]amine and 1.84 g (8.6 mmol) of 3-chloromethyl-5-phenyl-pyrazole hydrochloride in 7.5 ml of dimethyl formamide and the resulting reaction mixture was stirred at a temperature of 70°C for 16 hours.

After cooling, the reaction mixture was poured out on ice and extracted three times with ethyl acetate.

The collected organic layers were washed twice with dilute ammonia, dried on sodium sulphate and evaporated under reduced pressure.

The resulting crude product was purified chromatographically by means of flash chromatography over 300 g of silica gel (Merck, grain size 0.040-0.063 mm) using a mixture of dichloromethane, methanol and concentrated ammonia in the ratio 95.5:4.0:0.5 as an eluent.

After evaporating the collected fractions under reduced pressure, 2.4 g (6.9 mmol) of 4-[2-[propyl(5-phenylpyrazol-3-yl-methyl)amino]-2-methylethyl]phenol were obtained (compound 39).

Compound 40 was obtained in an analogous manner.

## Claims

1. Compounds of formula 1

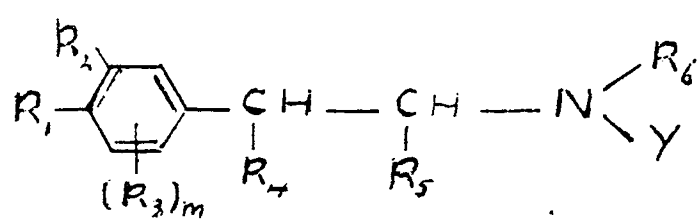

(1)

wherein:

$R_1$ is hydrogen, an optionally esterified hydroxyl group or mercapto group, a group $-NHR_9$ or $-CONHR_9$, wherein $R_9$ is hydrogen, alkyl having 1-6 C-atoms or alkylcarbonyl having 1-7 C-atoms;

$R_2$ is hydrogen or, when $R_1$ is hydrogen, may have one of the other meanings of $R_1$, or

$R_1$ and $R_2$ together with the 2 carbon atoms of the benzene ring constitute a heterocyclic group which consists of five or six ring atoms and which comprises a group -NH-and, optionally may comprise an oxygen atom, sulphur atom or nitrogen atom as a second hetero atom;

$R_3$ is hydrogen, alkyl, alkoxy or alkylthio having 1-4 C-atoms, amino, mono-or dialkylamino having 1-4 C-atoms per alkyl group, hydroxyalkyl, alkyl-, alkylamino-or alkoxycarbonyl having 1-4 C-atoms in the alkyl group, nitro, cyano, halogen, trifluoromethyl, trifluoromethoxy, alkylsulphonyl having 1-4 C-atoms, or aminosulphonyl;

m has the value 1, 2 or 3;

$R_4$ is hydrogen, alkyl or alkoxy having 1-3 C-atoms, or hydroxyl;

$R_5$ is hydrogen; alkyl, phenylalkyl, hydroxyalkyl, methoxyalkyl, alkylcarbonyl, alkoxycarbonyl or alkylaminocarbo nyl having 1-6 C-atoms in the optionally branched alkyl group;

$R_6$ is straight or branched alkyl, alkenyl or cycloalkylalkyl or cycloalkyl, having at most 8 C-atoms;

Y is a group $R_7$-X-$R_8$, wherein $R_7$ is a straight or branched alkylene chain having 3-8 C-atoms with at least 3 C-atoms between the nitrogen atom and group X; X is the carbonyl group or ketalised carbonyl group, or the group >CHOH, >$CHC_6H_5$, >$CH_2$, -CONH-or -CO- N $CH_3$ or an oxygen atom or sulphur atom; and $R_8$ is an alkyl group, cycloalkyl group or cycloalkylalkyl group having at most 10 C-atoms, a phenyl group or phenylalkyl group having 1-4 C-atoms in the alkyl group, which groups $R_8$ can be substituted with one or more groups $R_3$; or Y is a group of the formula 2a-2e

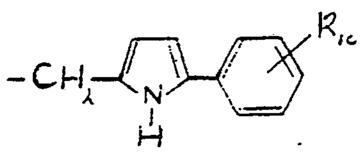

2a

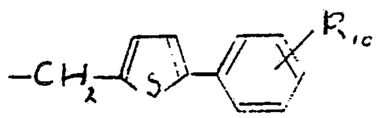

2b

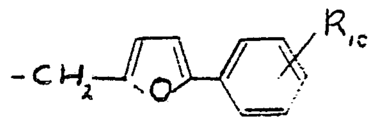

2c

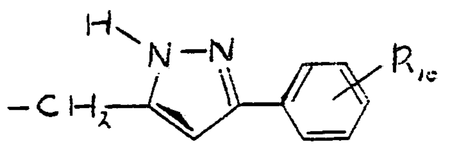

2d

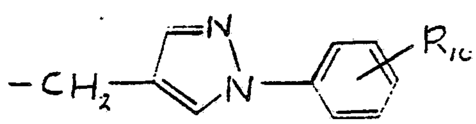

2e

wherein $R_{10}$ may have the meanings given for $R_3$, prodrugs and salts thereof.

2. Compounds as claimed in Claim 1 of the formula 1, wherein

$R_1$ is hydrogen, optionally esterified hydroxyl or aminocar bonyl;

$R_2$ is hydrogen, or, when $R_1$ is hydrogen, optionally esterified hydroxyl or aminocarbonyl;

$R_3$ is hydrogen, methyl, methoxy or halogen in the ortho position with respect to the group -$CHR_4$-$CHR_5$-$NR_6Y$;

m has the value 1;

$R_4$ is hydrogen or hydroxyl;

$R_5$ is alkyl having 1-3 C-atoms or phenyl ethyl;

$R_6$ is alkyl having 1-4 C-atoms, propenyl, butenyl or cyclopropylmethyl;

Y is the group $R_7$-X-$R_8$, wherein $R_7$ is trimethylene, X is carbonyl, >CHOH, -CONH-, -$CH_2$-or an oxygen atom, and $R_8$ is cyclohexyl, phenyl or halogen-substituted phenyl; or Y is a group of formula 2a, 2d or 2e, wherein $R_{10}$ is hydrogen or halogen; and prodrugs and salts thereof.

3. A method of preparing tertiary arylethylamine derivatives, characterized in that compounds as claimed in Claim 1 are prepared in a manner known per se.

4. A method as claimed in Claim 3, characterized in that compounds of formula 1, wherein Y is the group -$R_7$ -X - $R_8$, are prepared by reaction of a compound of formula 3

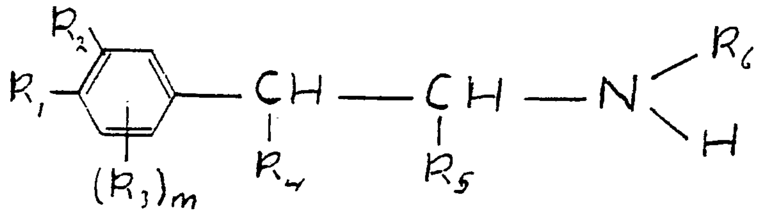

(3)

with a compound of the formula L - $R_7$ -X' -$R_8$ (4), wherein $R_1$ -$R_8$ and m have the meanings mentioned in Claim 1, L is a halogen atom or a tosyloxy group and X' is carbonyl, 1,3-dioxolane, CH-phenyl, -$CH_2$-or an oxygen atom or a sulphur atom.

5. A method as claimed in Claim 3, characterized in that compounds of formula 1, wherein Y is the group - $R_7$ -X - $R_8$, wherein X is the group -CONH-or -CO-N($CH_3$)-, are prepared by reaction of an ester of the formula 5

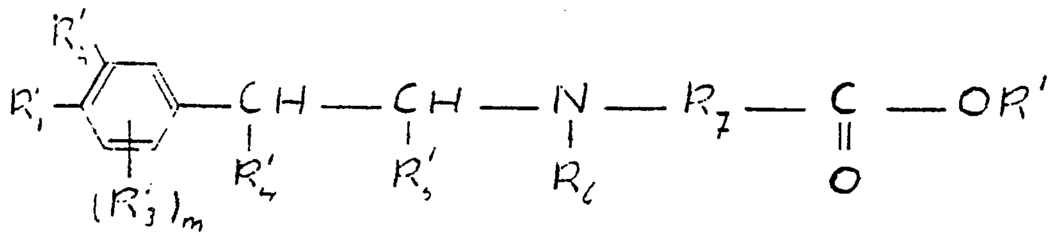

(5)

with an amine of the formula 6 or 7

$H_2N - R_8$ (6) $CH_3NH$-$R_8$ (7)

in which formulae $R_1'$ -$R_5'$ have the meanings mentioned in Claim 1 for $R_1$ -$R_5$, with the proviso that reactive hydrogen atoms present therein are replaced by a protective group, $R_6$ -$R_8$ and m have the meanings mentioned in Claim 1, and the protective group(s) are then removed in a manner known per se.

6. A method as claimed in claim 3, characterized in that compounds of formula 1, wherein Y is a a group of the formula 2a, 2b or 2c, are prepared by means of a Mannich reaction of an amine of formula 3, formaldehyde and a 2-phenylpyrrole or 2-phenylthiophene or 2-phenylfuran derivative.

7. A method as claimed in Claim 3, characterized in that compounds of formula 1, wherein Y is a group of formula 2d or 2e, are prepared by converting a compound of formula 3 with a compound of formula L-Y (10), wherein L is a halogen atom or a tosyloxy group and Y is a group of the formula 2d or 2e.

8. A method as claimed in Claim 3, characterized in that a compound of formula 1 is prepared by converting one or more of the groups $R_1$ -$R_{10}$, X and Y in a resulting compound of formula 1 into another group $R_1$ -$R_{10}$, X and Y with the meanings mentioned in Claim 1.

9. Pharmaceutical composition which comprise at least one compound as claimed in Claim 1 as the active substance.

10. A method of preparing pharmaceutical compositions having opiate-antagonistic activity, characterized in that a compound as claimed in Claim 1 is brought into a form suitable for administration.

European Patent Office

# EUROPEAN SEARCH REPORT

Application Number

EP 88 20 0648

## DOCUMENTS CONSIDERED TO BE RELEVANT

| Category | Citation of document with indication, where appropriate, of relevant passages | Relevant to claim |
|---|---|---|
| Y | GB-A- 586 645 (WELLCOME)<br>* Claims; page 7 * | 1,9,10 |
| X | GB-A- 587 244 (WELLCOME)<br>* Claims; page 5 * | 1,9,10 |
| X | NL-A-8 300 392 (DUPHAR)<br>* Claims * | 1,9,10 |
| X | EP-A-0 100 569 (DUPHAR)<br>* Claims * | 1,9,10 |
| Y | EP-A-0 211 254 (DEGUSSA)<br>* Claims * | 1,9,10 |
| Y | US-A-4 337 207 (M. GOODMAN)<br>* Claims * | 1,9,10 |
| X | FR-A-1 477 040 (DEGUSSA)<br>* Claims; page 1 * | 1,9,10 |
| X | FR-A-2 266 495 (PHILIPS)<br>* Claims * | 1,9,10 |
| X | FR-A-2 007 623 (DEGUSSA)<br>* Claims * | 1,9,10 |
| X | US-A-4 097 500 (S.S. PELOSI, Jr.)<br>* Claims * | 1,9,10 |
| D,X | GB-A-1 500 434 (PHILIPS)<br>* Claims * | 1,9,10 |

CLASSIFICATION OF THE APPLICATION (Int. Cl.4)

C 07 C 91/30
C 07 C 97/00
C 07 C 103/50
C 07 D 207/335
C 07 D 231/12
A 61 K 31/135
A 61 K 31/16
A 61 K 31/40
A 61 K 31/415

TECHNICAL FIELDS SEARCHED (Int. Cl.4)

C 07 C 91/00
C 07 C 97/00
C 07 C 103/00
C 07 D 201/00
C 07 D 231/00
A 61 K 31/00

The present search report has been drawn up for all claims

| Place of search | Date of completion of the search | Examiner |
|---|---|---|
| THE HAGUE | 22-07-1988 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)